(19) Europäisches Patentamt
European Patent Office
Office européen des brevets

(11) Publication number: **0 243 971 B1**

(12) **EUROPEAN PATENT SPECIFICATION**

(45) Date of publication of patent specification: **03.08.94**

(51) Int. Cl.5: **C07D 213/82**, C07D 213/83, A01N 53/00

(21) Application number: **87106296.4**

(22) Date of filing: **30.04.87**

The file contains technical information submitted after the application was filed and not included in this specification

(54) **Fungicidal pyridyl cyclopropane carboxamides.**

(30) Priority: **02.05.86 US 859152**
**23.03.87 US 29116**
**15.04.87 US 36542**

(43) Date of publication of application:
**04.11.87 Bulletin 87/45**

(45) Publication of the grant of the patent:
**03.08.94 Bulletin 94/31**

(84) Designated Contracting States:
**AT BE CH DE ES FR GB GR IT LI NL SE**

(56) References cited:
**EP-A- 243 970**
**GB-A- 1 078 288**
**GB-A- 2 056 974**
**EP-A- 0 190 036**
**GB-A- 2 001 316**
**US-A- 3 493 555**

(73) Proprietor: **STAUFFER CHEMICAL COMPANY**

**Westport Connecticut 06880(US)**

(72) Inventor: **Baker, Don Robert**
**15 Muth Drive**
**Orinda California 94563(US)**
Inventor: **Brownell, Keith Harvey**
**4751 Elmhurst Drive**
**San Jose California 95129(US)**

(74) Representative: **Jaeger, Klaus, Dr. et al**
**Jaeger, Böck & Köster,**
**Patentanwälte,**
**Postfach 16 20**
**D-82121 Gauting (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The invention relates to fungicidal pyridyl cyclopropane carboxamides, to a fungicidal composition comprising said carboxamides and to a method of controlling fungi.

Fungal infection of crops such as barley, rice, tomatoes, wheat, beans, roses, grapes and other agriculturally important crops can cause heavy losses in both quantity and quality of agricultural products. It is therefore extremely desirably to have means of preventing, controlling or eliminating fungal growth. Much preventive spraying with commercial fungicides is conducted to attempt to prevent the establishment and growth of fungi on agriculturally important crops. It would also be desirable to have a curative fungicide which, on detection of fungal infestation, could destroy the fungi and eliminate the deleterious effects by use of a postinfestation curative spray.

GB-A-1 078 288 discloses pyridyl amides and in particular N-(2-methoxy-5-pyridyl)-isobutyramide. Said known compounds shall be active as fungicides.

Furthermore US-A-3 493 555 teaches pyridyl cyclopropane carboxamides being active as herbicides. Heterocyclic acylamino compounds having fungicidal, herbicidal and plant-growth regulating properties are described in GB-A-2 056 974.

The present invention provides novel fungicidal pyridyl cyclopropane carboxamides having the following structural formula:

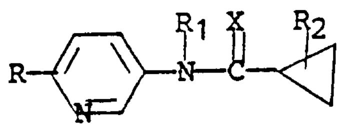

wherein R is selected from the group of -Cl and -$OCH_3$,
$R_1$ is selected from the group of -H and -C≡N,
$R_2$ is selected from the group of -H, 1-$CH_3$ and 2-$CH_3$, and
X is O,
and the fungicidally acceptable organic and inorganic salts thereof.

The compounds described above and the organic and inorganic salts thereof are highly effective fungicides for use both as preventive and curative fungicides.

Provided are furthermore a fungicidal composition comprising a compound as described above and a method of controlling fungi comprising applying to the area where control is desired a compound as described above.

The term "fungicide" is used to mean a compound which controls fungal growth. "Controls" includes prevention, destruction and inhibition of fungal growth. The term "curative" is meant to refer to a post-infection application of a fungicide which establishes control of fungal infection and prevents development of deleterious effects of the fungi on the host crop.

The compounds of this invention can be generally prepared by reacting a properly substituted 5-aminopyridine with a properly substituted cyclopropane carboxylic acid chloride in a polar solvent such as dichloromethane in a suitable reactor. It is desirable to maintain an acid scavenger such as pyridine in the reaction vessel. The reaction generally will proceed at room temperature but will operate at a temperature range from -30 to 60 °C, depending on the substitutions on the amino pyridine and the cyclopropane carboxylic acid chloride. The reaction should go to completion within 1 to 3 hours. The resulting product is recovered in a conventional manner by washing with an alkali solution such as NaOH and water, drying over conventional drying agents such as magnesium sulfate, and crystallizing in hexane.
Salts of the various pyridyl cyclopropane carboxamides can be conventionally prepared by reacting at least a molar amount of a Lewis acid with the carboxamide. Preferably the reaction is run in a solvent for the carboxamide with heating if necessary. The prepared salt is recovered from the reaction mixture by conventional techniques.

Pyridyl carboxamides of the invention are basic. The unprotonated nitrogen atom of the pyridyl ring can be protonated by an acid, either organic or inorganic. Representative inorganic acids are hydrochloric, nitric, hydrobromic, sulfuric, sulfamic and phosphoric. Representative organic acids are acetic, trifluoroacetic, benzoic, benzenesulfonic, p-toluenesulfonic, naphthalenesulfonic, phenylphosphonic and organophosphonic. The salts so formed are also fungicidal.

EXAMPLE 1

Preparation of N-(2-Methoxy-5-pyridyl)-cyclopropane carboxamide

5-Amino-2-methoxy pyridine (12.4 grams, 0.10 mole) and 10 milliliters (ml) of pyridine are mixed together in 200 ml dichloromethane in a reaction flask. 9.1 ml (0.10 mole) of cyclopropane carboxylic acid chloride was added to the reaction mixture over a period of 2 minutes. The reaction was exothermic and temperature rose to 34°C. The reaction was allowed to stand for one hour at room temperature, after which the reaction mixture was washed with 200 ml of 5% sodium hydroxide and 100 ml of water. The resulting organic phase was separated and dried over anhydrous magnesium sulfate. Crystals formed, so the mixture was filtered and washed with 300 ml of acetone and the filtrate evaporated in vacuo to give a solid that was triturated with hexane to yield 16.7 grams, after drying. The product was identified by IR and NMR as the title compound, having a melting point of 130-131°C. This compound will be referred to as Compound 1.

EXAMPLE 2

Preparation of N-(2-Methoxy-5-pyridyl)-cyclopropane carboxamide dodecanoic acid salt

Three grams (0.016 mole) of the compound of Example 1 was dissolved in 100 ml of acetone in a 300 ml, one-neck, round-bottom flask by swirling. 3.2 g (0.016 mole) dodecanoic acid was added which went into solution. The resulting solution was evaporated on a rotary evaporator, yielding 6.2 g, of a white solid having a melting point of 92-96°C, which was identified by nuclear magnetic resonance spectroscopy as the title compound. This compound will be known as Compound 7.

Representative compounds of this invention and their physical properties are shown in Table I.

TABLE I

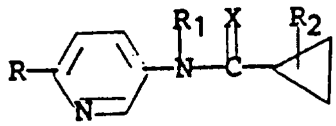

| Cmpd. No. | R | R1 | R2 | X | $n_D^{30}$ or melting point °C |
|---|---|---|---|---|---|
| 1 | $-OCH_3$ | -H | -H | O | 130.0 - 131.0 |
| 2 | -Cl | -H | -H | O | 167.0 - 169.0 |
| 3 | $-OCH_3$ | -C≡N | -H | O | 84.0 - 92.0 |
| 4 | $-OCH_3$ | -H | $1-CH_3$ | O | 70.0 - 74.0 |
| 5 | $-OCH_3$ | -H | $2-CH_3$ | O | 66.0 - 70.0 |
| 6 | hydrochloric acid salt of Cmpd. 1 | | | | 144.0 - 146.0 |
| 7 | dodecanoic acid salt of Cmpd. 1 | | | | 92.0 - 96.0 |
| 8 | 2-naphthalene-sulfonic acid salt of Cmpd. 1 | | | | 90.0 - 96.0 |
| 9 | acetic acid salt of Cmpd. 1 | | | | 112.0 - 120.0 |
| 10 | benzoic acid salt of Cmpd. 1 | | | | 78.0 - 85.0 |
| 11 | chloromethyl-phosphonic acid salt of Cmpd. 1 | | | | 110.0 - 118.0 |
| 12 | hydrochloric acid salt of Cmpd. 2 | | | | 134.0 - 136.0 |
| 13 | phenyl phosphonic acid salt of Cmpd. 1 | | | | 108.0 - 115.0 |
| 14 | sulfamic acid salt of Cmpd. 1 | | | | 131.0 - 136.0 |
| 15 | trifluoroacetic acid salt of Cmpd. 1 | | | | 89.0 - 93.0 |
| 16 | nitric acid salt of Cmpd. 1 | | | | 144.0 - 145.0 |

EXAMPLE 3

Mole Equivalent Preventative Spray Evaluation Procedures

Barley Powdery Mildew (PM)

Northrup King Sunbar 401 barley seed is planted (12 seeds/2" pot) in a sandy-loam soil seven days prior to testing. The test compound is diluted in a 50/50 acetone/water solution to produce concentrations decreasing from 0.004 molar. The test solution is then sprayed onto the barley plants with atomizing sprayers.

Twenty-four hours later, test plants are placed in an inoculation box equipped with a circulating fan. Barley plants with heavily sporulating *Erysiphe graminis* lesions are placed in front of the fan to dislodge and distribute the spores. After two minutes the fan is shut off and the chamber is left closed five minutes for the spores to settle. Inoculated plants are teen placed on an automatic sub-irrigation greenhouse bench.

Results are recorded seven days following inoculation as percent disease control based on the percent reduction in lesion area as compared to the untreated control plants. Compound concentrations which provide 90% disease control (CO 90) are determined from dosage/dilution curves.

Leaf Rust (LR)

Seven seeds of Anza wheat are planted in 2" pots in a sandy-loam soil 12 days prior to testing. The compound to be tested is diluted with a 50/50 acetone/water solution to produce concentrations decreasing from 0.004 molar. Twelve ml of test solution are sprayed onto the wheat plants with an atomizing sprayer.

A suspension of Puccinia recondita urediospores is prepared by vacuuming spores from wheat leaves with ureida pustules and suspending $10^5$ spores/ml in deionised water plus 0.5% Tween® 20 (polyoxyethylene sorbitan monolaurate). Plants are inoculated 24 hours after treatment by spraying with the spore suspension to runoff, allowing it to dry on the leaves, respraying to runoff, and then placing the plants into a dark mist chamber. Following 48 hours in the mist, plants are moved to a subirrigation greenhouse bench.

Results are recorded ten days following inoculation as percent disease control based on the percent reduction in lesion area as compared to the untreated control plants. Compound concentrations which provide 90% disease control (EC 90) are determined from dosage/dilution curves.

Botrytis Bud Blight (BB)

Two white rose petals are placed in a petri dish lined with wet filter paper. The compound to be tested is diluted with a 50/50 acetone/water solution to produce concentrations decreasing from 0.004 molar. On half ml of test solution is atomized onto the petals, and allowed to dry.

Inoculum is prepared by adding a 5 mm plug from a two-week old Botrytis cineria culture grown on Elliot's V-8 agar, to 10 ml sterile distilled water plus 0.5% grape juice. A 20 ul drop of this inoculum suspension is placed on each petal. Petri dishes with inoculated petals are stored in sealed plastic boxes to maintain saturated humidity.

Results are read four days following inoculation as a percent reduction in necrotic area compared to the acetone/water controls. Compound concentrations which provide 90% disease control (EC 90) are determined from dosage/dilution curves.

The molar equivalent of 0.004 used as the initial dilution in these evaluation procedures is equivalent to 750 parts per million (ppm) of the amides. All salts tested were adjusted in a solution to 0.004 molar which is equivalent to the 750 ppm of the amide from which the salt was made.

The results are presented in Table II as an approximate EC 90 in parts per million. The entry (750) indicates partial control at 750 ppm.

TABLE II

| Cmpd. No. | PM | LR | BB |
|---|---|---|---|
| 1 | 200 | 200 | 20 |
| 2 | 250 | 750 | 25 |
| 3 | (750) | 500 | 25 |
| 4 | >750 | >750 | 750 |
| 5 | (750) | 200 | 200 |
| 6 | 200 | 200 | 70 |
| 7 | 200 | 200 | 20 |
| 8 | 200 | 200 | 20 |
| 9 | 200 | 200 | 20 |
| 10 | 200 | 200 | 20 |
| 11 | 200 | 200 | 20 |
| 12 | - | - | 80 |
| 13 | 200 | 200 | 20 |
| 14 | - | - | 20 |
| 15 | - | - | 20 |
| 16 | - | - | 20 |

EXAMPLE 4

Post-Infection Fungicide Screening Procedures

Apple Scab (AS)

Apple seedlings are inoculated by spraying the foliage with Venturia inaequalis using standard procedures 24 hours. Following inoculation the test compound is applied in solution as a foliage spray at the rate of 160 mg/l. Results are determined after sympton development from the reduction in lesion area as compared to the untreated control plants.

Rice Blast (RB)

Six days before inoculation, seedling rice plants are drenched with a solution containg 50 mg test compound/liter of soil. Inoculation with Piricularia oryzae is performed using standard procedures. Results are determined after symptom development from the reduction in lesion area as compared to the untreated control plants.

Wheat Blume Blotch (GB)

Two days before inoculation, the test compound is applied in solution to wheat seedlings as a foliage spray at the rate of 500 mg/l. Inoculation with Septoria nodorum is performed using standard procedures. Results are determined after sympton development from the reduction in lesion area as compared to the untreated control plants.

Wheat Foot Rot (FR)

wheat seed infested with Fusarium culmorum is treated with 500 mg/Kg of test compound using standard methods. Results are determined after symptom development from the reduction in disease as compared to the untreated control plants.

Activity is reported on the following basis: O - nil; X = light; XX = moderate and XXX = high.

TABLE III

| Cmpd. No. | Disease | | | |
|---|---|---|---|---|
| | AS | RB | GB | FR |
| 1 | XXX | X | XX | X |
| 2 | O | X | O | X |

Example 5

CURATIVE SPRAY EVALUATION PROCEDURES

Leaf Rust (LR)

Seven seeds of Anza wheat are planted in 2" pots in a sandy loam soil 12 days prior to testing. A suspension of Puccinia recondita urediospores is prepared by vacuuming spores from wheat leaves with uredia pustules and suspending $10^5$ spores/ml in deionized water plus 0.5% Tween® 20 (polyoxyethylene sorbitan monolaurate). Plants are inoculated by spraying with the spore suspension to runoff, allowing it to dry on the leaves, respraying to runoff, and then placing the plants into a mist chamber. Following 48 hours in the mist, plants are moved to a subirrigation greenhouse bench.

The compound to be tested is diluted in a 50/50 acetone/water solution to produce concentrations decreasing from 0.075%. Fifty hours following inoculation, the plants are placed on a rotating turntable and sprayed with the test solution to near runoff with atomizing nozzles. (Time of inoculation is defined as when plants are placed into the mist chamber.)

Results are recorded ten days following inoculation as percent disease control based on the percent reduction in lesion area as compared to the untreated control plants.

TABLE III

| Cmpd. No. | LR |
|---|---|
| 1 | 100 |
| 2 | 100 |
| 3 | 100 |
| 4 | 100 |
| 5 | 100 |
| 6 | 100 |
| 7 | 100 |
| 8 | 100 |
| 9 | 100 |
| 10 | 100 |
| 11 | 100 |

The compounds of this invention are particularly effective against Botrytis Bud Blight and are particularly effective as preventative foliar sprays and curative foliar sprays when compared to standard commercial compounds used as Botrytis preventative and curative sprays. Fungi on which the compounds of the present invention are particularly effective are as follows: Botrytis cinerea; Venturia inaequalis; Septoria nodorum; Erysiphe graminis; Fusarium culmorum; Piricularia oryzae; and Puccinia graminis.

The compounds of the present invention are useful as fungicides, especially as preventative or curative fungicides, and can be applied in a variety of ways at various concentrations. In general, these compounds and formulations of these compounds can be applied directly to the crop foliage, the soil in which the crop is growing, or in the irrigation water for the crop or soil. In practice, the compounds herein defined are formulated into fungicidal compositions, by admixture, in fungicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active fungicidal compounds may be formulated as wettable powders, as emulsifiable concentrates, as powdery dusts, as solutions or as any of several other known types of formulations, depending upon the desired mode of application. Preferred formulations for preventative or curative fungicidal applications are wettable powders and emulsifiable concentrates. These formulations may contain as little as about 0.1% to as much as about 95% or more by weight of active ingredient. A fungicidally effective amount depends upon the nature of the seeds or plants to be treated and the rate of application varies from about 0.05 to approximately 25 pounds per acre, preferably from about 0.1 to about 10 pounds per acre.

Wettable powders are in the form of finely divided particles which disperse readily in water or other dispersants. The wettable powder is ultimately applied to the soil or plant either as a dry dust or as a dispersion in water or other liquid. Typical carriers for wettable powders include fuller's earth, kaolin clays, silicas and other readily wet organic or inorganic diluents. Wettable powders normally are prepared to contain about 5% to about 95% of the active ingredient and usually also contain a small amount of wetting, dispersing, or emulsifying agent to facilitate wetting and dispersion.

Dry flowables or water dispersible granules are agglomerated wettable powders made by either pan granulation or by fluidized bed. The dry flowable is ultimately applied to the soil or plant as a dispersion in water or other liquid. These granules are dust-free and free flowing when dry and yet upon dilution in water, form homogeneous dispersions. Typical carriers for dry flowables include fuller's earth, kaolin clays, silicas and other readily wet organic or inorganic diluents. The dry flowables normally are prepared to contain from about 5% to about 95% of the active ingredient and usually contain a small amount of wetting, dispersing or emulsifying agent to facilitate wetting and dispersion.

Emulsifiable concentrates are homogeneous liquid compositions which are dispersible in water or other dispersant, and may consist entirely of the active compound with a liquid or solid emulsifying agent, or may also contain a liquid carrier, such as xylene, heavy aromatic naphtha, isophorone and other non-volatile organic solvents. For fungicidal application, these concentrates are dispersed in water or other liquid carrier and normally applied as a spray to the area to be treated. The percentage by weight of the essential active ingredient may vary according to the manner in which the composition is to be applied, but in general

comprises about 0.1% to 95% of active ingredient by weight of the fungicidal composition.

Typical wetting, dispersing or emulsifying agents used in agricultural formulations include, for example, the alkyl and alkylaryl sulfonates and sulfates and their sodium salts; polyhydroxy alcohols; and other types of surface-active agents, many of which are available in commerce. The surface-active agent, when used, normally comprises from 0.1% to 15% by weight of the fungicidal composition.

Dusts, which are free-flowing admixtures of the active ingredient with finely divided solids such as talc, clays, flours and other organic and inorganic solids which act as dispersants and carriers for the toxicant, are useful formulations for many applications.

Pastes, which are homogeneous suspensions of a finely divided solid toxicant in a liquid carrier such as water or oil, are employed for specific purposes. These formulations normally contain about 5% to about 95% of active ingredient by weight, and may also contain small amounts of a wetting, dispersing or emulsifying agent to facilitate dispersion. For application, the pastes are normally diluted and applied as a spray to the area to be affected.

## EXAMPLES OF TYPICAL FORMULATIONS

**Oil**

| Ingredient | Weight % |
|---|---|
| Compound 1 | 1 |
| Oil solvent-heavy aromatic naphtha | 99 |
| Total | 100 |

**Emulsifiable Concentrate**

| Ingredient | Weight % |
|---|---|
| Compound 2 | 50 |
| Kerosene | 45 |
| Emulsifying agent (mixture of long chain ethoxylated polyethers with long chain sulfonate) | 5 |
| Total | 100 |

**Emulsifiable Concentrate**

| Ingredient | Weight % |
|---|---|
| Compound 3 | 90 |
| Kerosene | 5 |
| Emulsifying agent (mixture of long chain ethoxylated polyethers with long chain sulfonate) | 5 |
| Total | 100 |

**Dusts and/or Powders**

| Ingredient | Wt. % | Wt. % | Wt. % |
|---|---|---|---|
| Compound 4 | 0.5 | 50.0 | 90.0 |
| Attapulgite Clay Powder | 93.5 | 44.0 | 4.0 |
| Sodium lignin sulfonate | 5.0 | 5.0 | 5.0 |
| Sodium dioctyl sulfosuccinate | 1.0 | 1.0 | 1.0 |
| Total | 100.0 | 100.0 | 100.0 |

Other useful formulations for fungicidal applications include simple solutions of the active ingredient in a dispersant in which it is completely soluble at the desired concentration, such as acetone, alkylated naphthalenes, xylene and other organic solvents. Pressurized sprays, typically aerosols, wherein the active ingredient is dispersed in finely divided form as a result of vaporization of a low boiling dispersant solvent carrier, such as the Freons®, may also be used.

The fungicidal compositions of this invention are applied to the plants in the conventional manner. Thus, the dust and liquid compositions can be applied to the plant by the use of power-dusters, boom and hand

sprayers and spray dusters. The compositions can also be applied from airplanes as a dust or a spray because they are effective in low dosages.

**Claims**

**1.** A compound having the following structural formula

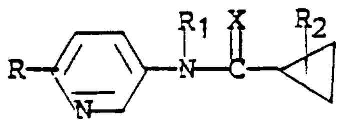

wherein R is selected from the group of -Cl and $-OCH_3$,
$R_1$ is selected from the group of -H and -C≡N,
$R_2$ is selected from the group of -H, $1-CH_3$ and $2-CH_3$, and
X is O,
and the fungicidally acceptable organic and inorganic salts thereof.

**2.** The compound of Claim 1 wherein R is $-OCH_3$, $R_1$ is -H and $R_2$ is -H.

**3.** The compound of Claim 1 wherein R is -Cl, $R_1$ is -H and $R_2$ is -H.

**4.** The compound of Claim 1 wherein R is $-OCH_3$, $R_1$ is -C≡N and $R_2$ is -H.

**5.** A fungicidal composition comprising a compound as claimed in one of the Claims 1 to 4 and an inert diluent carrier therefor.

**6.** A method of controlling fungi comprising applying to the area where control is desired a compound as claimed in one of the Claims 1 to 4.

**Patentansprüche**

**1.** Verbindungen der folgenden allgemeinen Strukturformel

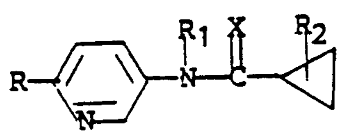

worin R ausgewählt ist aus der Gruppe bestehend aus -Cl und $-OCH_3$,
$R_1$ ausgewählt ist aus der Gruppe bestehend aus -H und -C≡N,
$R_2$ ausgewählt ist aus der Gruppe bestehend aus -H, $1-CH_3$ und $2-CH_3$ und
X für O steht,
und die fungizid akzeptierbaren organischen und anorganischen Salze davon.

**2.** Verbindung nach Anspruch 1, worin R für $-OCH_3$, $R_1$ für -H und $R_2$ für -H stehen.

**3.** Verbindung nach Anspruch 1, worin R für Cl, $R_1$ für -H und $R_2$ für -H stehen.

**4.** Verbindung nach Anspruch 1, worin R für $-OCH_3$, $R_1$ für -C≡N und $R_2$ für -H stehen.

**5.** Fungizide Zusammensetzung enthaltend eine Verbindung nach einem der Ansprüche 1 bis 4 und einen inerten verdünnenden Träger dafür.

**6.** Verfahren zur Kontrolle von Pilzen, wobei man auf dasjenige Gebiet, welches man zu kontrollieren wünscht, eine Verbindung nach einem der Ansprüche 1 bis 4 aufbringt.

**Revendications**

**1.** Un composé présentant la formule structurelle suivante:

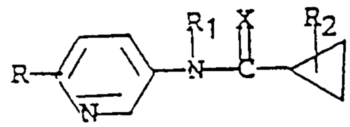

dans laquelle:

R est sélectionné dans le groupe comprenant -Cl et $-OCH_3$;

$R_1$ est sélectionné dans le groupe comprenant -H et -C≡N;

$R_2$ est sélectionné dans le groupe comprenant -H, $1-CH_3$ et $2-CH_3$; et

X représente O,

ainsi que les sels organiques et inorganiques, acceptables du point de vue fongicide, dérivant de ce composé.

**2.** Le composé selon la revendication 1, dans lequel:

R représente $-OCH_3$;

$R_1$ représente -H; et

$R_2$ représente -H.

**3.** Le composé selon la revendication 1, dans lequel:

R représente Cl;

$R_1$ représente -H; et

$R_2$ représente -H.

**4.** Le composé selon la revendication 1, dans lequel:

R représente $-OCH_3$;

$R_1$ représente -C≡N; et

$R_2$ représente -H.

**5.** Une composition fongicide comprenant un composé tel que revendiqué dans l'une des revendications 1 à 4, ainsi qu'un support diluant inerte de ce dernier.

**6.** Un procédé de contrôle du développement des champignons comprenant l'application sur la surface où le contrôle est souhaité d'un composé tel que revendiqué dans l'une des revendications 1 à 4.